# EUROPEAN PATENT APPLICATION

(11) **EP 4 498 045 A1**
(43) Date of publication of application: **29.01.2025**
(21) Application number: 24185232.6
(22) Date of filing: 28.06.2024
(51) Int. Cl.: G01F 1/7086, G01F 1/74, A61M 1/36

(54) **APPARATUS AND METHOD FOR BUBBLE DETECTION IN FLUID FLOW**

(30) Priority: 28.07.2023 IN 202311051004
(71) Applicant: Honeywell International Inc., Charlotte, NC 28202 (US)
(72) Inventor: KISHORE, Kuna Venkat Satya Rama, Charlotte, 28202 (US); KUMAR, Mithlesh, Charlotte, 28202 (US); G V, Vinay Kumar, Charlotte, 28202 (US)
(74) Representative: Haseltine Lake Kempner LLP

(57) **Abstract**

A bubble detection device is provided. For example, a bubble detection device comprises a light source, a light sensor, and one or more processors. The light source is adapted to be positioned to shine light through a tube through which a fluid flows. The light sensor is adapted to be positioned on an opposite side of the tube from the light source and to receive light from the light source transmitted through the tube. The processors (i) detect a first motion across the light sensor corresponding to a leading edge of a bubble in the fluid, (ii) detect a second motion across the light sensor corresponding to a trailing edge of the bubble in the fluid, and (iii) calculate a length of the bubble based on (a) a velocity of the bubble and (b) a time between detection of the first motion and detection of the second motion.

## Description

### FIELD OF THE INVENTION

Embodiments of the present disclosure generally relate to imaging systems, and, more particularly, to imaging systems for bubble detection.

### BACKGROUND

In infusion pumps, drug delivery devices, and the like, fluid flow breaks and air bubbles in the fluid delivery tube may be harmful to a patient's health. Similarly, in industrial applications, breaks in fluid flow to industrial devices, such as mixers, separators, etc., may be disruptive to the industrial process and potentially damaging to the industrial devices.

It is known to use bubble detection devices to detect such bubbles and breaks in the fluid flow. However, such bubble detection devices are plagued by technical challenges and limitations. Through applied effort, ingenuity, and innovation, many of these identified problems have been solved by developing solutions that are included in embodiments of the present disclosure, many examples of which are described in detail herein.

### BRIEF SUMMARY

Various embodiments described herein relate to bubble detection devices and associated methods of detecting a bubble in a fluid flowing through a tube.

In accordance with various embodiments of the present disclosure, a bubble detection device is provided. In some embodiments, the bubble detection device comprises a light source, a lights sensor, and one or more processors. The light source is adapted to be positioned to shine light through a tube through which a fluid flows. The light sensor is adapted to be positioned on an opposite side of the tube from the light source and to receive light from the light source transmitted through the tube. The one or more processors (i) detect a first motion across the light sensor, the first motion corresponding to a leading edge of a bubble in the fluid, (ii) detect a second motion across the light sensor, the second motion corresponding to a trailing edge of the bubble in the fluid, and (iii) calculate a length of the bubble based on (a) a velocity of the bubble and (b) a time between detection of the first motion and detection of the second motion.

In some embodiments, the light is received by the light sensor and processed by the one or more processors at a frame refresh rate.

In some embodiments, the one or more processors calculate a velocity of the bubble based on an amount of movement of the bubble across the light sensor in one frame multiplied by the frame refresh rate.

In some embodiments, the one or more processors calculates a velocity of the bubble based on (1) an amount of movement of the leading edge across the light sensor in the detected first motion multiplied by the frame refresh rate, (2) an amount of movement of the trailing edge across the light sensor in the detected second motion multiplied by the frame refresh rate, or (3) an average of the amount of movement of the leading edge across the light sensor in the detected first motion multiplied by the frame refresh rate and the amount of movement of the trailing edge across the light sensor in the detected second motion multiplied by the frame refresh rate.

In some embodiments, the first motion is expressed as an X-axis motion vector and/or a Y-axis motion vector.

In some embodiments, the one or more processors detect a reverse flow of the fluid based on a negative value of the X-axis motion vector and/or a negative value of the Y-axis motion vector.

In some embodiments, the one or more processors start a timer when the first motion is detected and stop the timer when the second motion is detected.

In some embodiments, the one or more processors calculate an estimate of a size of the bubble based on 0.5 * (an amount of movement of the leading edge across the light sensor in the detected first motion + an amount of movement of the trailing edge across the light sensor in the detected second motion) * the length of the bubble.

In some embodiments, the one or more processors determine if a break in the fluid flow has occurred by determining if the bubble length is greater than a predetermined multiple of a diameter of the tube.

In some embodiments, the one or more processors determine if the bubble is classified as a small bubble by determining if the bubble length is less than a predetermined fraction of a diameter of the tube.

In accordance with various embodiments of the present disclosure, a method of detecting a bubble in a fluid flowing through a tube is provided. In some embodiments, the method comprises shining light from a light source positioned to shine the light through the tube; receiving, by a light sensor positioned on an opposite side of the tube from the light source, light from the light source transmitted through the tube; detecting, by one or more processors, a first motion across the light sensor, the first motion corresponding to a leading edge of a bubble in the fluid; detecting, by the one or more processors, a second motion across the light sensor, the second motion corresponding to a trailing edge of the bubble in the fluid; and calculating, by the one or more processors, a length of the bubble based on (a) a velocity of the bubble and (b) a time between detection of the first motion and detection of the second motion.

The foregoing illustrative summary, as well as other exemplary objectives and/or advantages of the disclosure, and the manner in which the same are accomplished, are further explained in the following detailed description and its accompanying drawings.

### BRIEF DESCRIPTION OF THE DRAWINGS

The description of the illustrative embodiments may be read in conjunction with the accompanying figures. It will be appreciated that, for simplicity and clarity of illustration, elements illustrated in the figures have not necessarily been drawn to scale, unless described otherwise. For example, the dimensions of some of the elements may be exaggerated relative to other elements, unless described otherwise. Embodiments incorporating teachings of the present disclosure are shown and described with respect to the figures presented herein, in which:
FIG. 1 is a perspective view of an example bubble detection device in accordance with example embodiments of the present disclosure;
FIG. 2 is a simplified sectional view of a portion of the example bubble detection device of FIG. 1;
FIG. 3 is a block diagram of an example bubble detection device in accordance with example embodiments of the present disclosure;
FIGS. 4A, 4B, and 4C illustrate example detections of, respectively, a typical bubble, a small bubble, and a break in the fluid flow; and
FIGS. 5A and 5B are a flowchart illustrating an example method of detecting a bubble in a fluid flowing through a tube in accordance with example embodiments of the present disclosure.

### DETAILED DESCRIPTION OF THE INVENTION

Some embodiments of the present disclosure will now be described more fully hereinafter with reference to the accompanying drawings, in which some, but not all embodiments of the disclosure are shown. Indeed, these disclosures may be embodied in many different forms and should not be construed as limited to the embodiments set forth herein; rather, these embodiments are provided so that this disclosure will satisfy applicable legal requirements. Like numbers refer to like elements throughout.

As used herein, terms such as "front," "rear," "top," "bottom," "left," "right," etc. are used for explanatory purposes in the examples provided below to describe the relative position of certain components or portions of components. Furthermore, as would be evident to one of ordinary skill in the art in light of the present disclosure, the terms "substantially" and "approximately" indicate that the referenced element or associated description is accurate to within applicable engineering tolerances.

As used herein, the term "comprising" means including but not limited to and should be interpreted in the manner it is typically used in the patent context. Use of broader terms such as comprises, includes, and having should be understood to provide support for narrower terms such as consisting of, consisting essentially of, and comprised substantially of.

The phrases "in one embodiment," "according to one embodiment," "in some embodiments," and the like generally mean that the particular feature, structure, or characteristic following the phrase may be included in at least one embodiment of the present disclosure and may be included in more than one embodiment of the present disclosure (importantly, such phrases do not necessarily refer to the same embodiment).

The phrases "in one example," "according to one example," "in some examples," and the like generally mean that the particular feature, structure, or characteristic following the phrase may be included in at least one example of the present disclosure and may be included in more than one example of the present disclosure (importantly, such phrases do not necessarily refer to the same example).

If the specification states a component or feature "may," "can," "could," "should," "would," "preferably," "possibly," "typically," "optionally," "for example," "as an example," "in some examples," "often," or "might" (or other such language) be included or have a characteristic, that specific component or feature is not required to be included or to have the characteristic. Such component or feature may be optionally included in some examples, or it may be excluded.

The word "example" or "exemplary" is used herein to mean "serving as an example, instance, or illustration." Any implementation described herein as "example" or "exemplary" is not necessarily to be construed as preferred or advantageous over other implementations.

The term "electronically coupled," "electronically coupling," "electronically couple," "in communication with," "in electronic communication with," or "connected" in the present disclosure refers to two or more elements or components being connected through wired means and/or wireless means, such that signals, electrical voltage/current, data and/or information may be transmitted to and/or received from these elements or components.

The term "component" may refer to an article, a device, or an apparatus that may comprise one or more surfaces, portions, layers and/or elements. For example, an example component may comprise one or more substrates that may provide underlying layer(s) for the component and may comprise one or more elements that may form part of and/or are disposed on top of the substrate. In the present disclosure, the term "element" may refer to an article, a device, or an apparatus that may provide one or more functionalities.

The term "bubble" is used herein to mean a globule of a gas substance in a fluid. The term "fluid break" is used herein to mean an air void in the fluid that is several times longer than the diameter of the tube.

There are many different devices for delivering, such as via one or more pumps and one or more lengths of tubing, a fluid. Some of these devices are used in the medical field. For example, infusion pumps deliver an intravenous (IV) fluid, often mixed with one or more liquid medications, via an IV tube and an IV catheter into a patient's bloodstream. If bubbles are inadvertently introduced into the IV tube, that can endanger the patient. For that reason, it is important to detect those bubbles so that appropriate corrective action can be taken.

In another example, a hemodialysis device pumps blood from a patient to a filter that removes waste from the blood, typically via a dialysate, and then pumps the blood back to the patient. It is important to detect bubbles in the blood flowing through the tubes. It is also important to detect the direction of the blood flow to ensure that the blood is flowing properly through the hemodialysis device.

Various embodiments of the present disclosure overcome the above technical challenges and difficulties and provide various technical improvements and advantages. For example, various embodiments of the present disclosure provide an example device for detecting bubbles in a fluid flowing through a tube. Various embodiments of the present disclosure provide an example device for detecting a break in fluid flowing through a tube. Various embodiments of the present disclosure provide an example device for detecting a direction of fluid flowing through a tube. Various embodiments of the present disclosure provide an example device and method for detecting bubbles in a fluid flowing through a tube which is not based on amount of light attenuated by the fluid or the bubble, but rather which leverages optical motion detection methods.

In some embodiments, a bubble detection device comprises an optical tracking sensor. Such optical tracking sensors have an array of individual sensing elements, such as in a 64x64 pixel arrangement or a 128x128 pixel arrangement. Each pixel of such optical tracking sensors has a size or pitch (for example, 50 micrometers). In some embodiments, such optical tracking sensors have a configurable frame refresh rate. In some embodiments the frame refresh rate may be set at, for example, 1000 Hertz (Hz) or 2000 Hz. In some embodiments, a higher frame refresh rate is generally better than a lower frame refresh rate. In some embodiments, an optical tracking sensor such as the PAA5100JE-Q from PixArt Imaging Inc. or the ADNS 3050-M from Avago Technologies Limited is used in such a bubble detection device.

In some embodiments, a bubble detection device comprises an optical tracking sensor that receives light transmitted through the tube via a light source positioned on an opposite side of the tube from the optical tracking sensor. In some embodiments, the light source is a light emitting diode (LED). In some embodiments, the LED is an infrared LED. In some embodiments, the LED is an infrared LED with a wavelength of around 820 to 980 nanometers (nm). In some embodiments, the LED is an infrared LED with a wavelength of around 950-980 nanometers. In some embodiments, the LED is a visible light LED, which may be suitable for industrial applications. In some embodiments, the LED is a visible light LED with a wavelength of about 450 to 525nm, 550 to 675nm, or 700 to 800nm. In some embodiments, the wavelength of the light emitted is selected such that the tube is transparent to the light. In some embodiments, the wavelength of the light emitted is selected for use with tubes constructed from polyvinyl chloride (PVC), polyethylene, thermoplastic elastomers (TPE), nylon, or silicone.

In some embodiments, a bubble detection device detects bubbles in tubes having a diameter of about 3 millimeters to about 25 millimeters. In some embodiments, a bubble detection device receives a portion of the tube in a slot. In some embodiments, a bubble detection device detects bubbles in tubes through which a fluid is flowing at typical flow rates used to deliver IV fluid, such as about 0.5 milliliter per hour to about 1 liter per hour.

In some embodiments, a bubble detection device detects a leading edge (LE) of a bubble or fluid break and/or a trailing edge (TE) of a bubble or fluid break. The process of detecting a bubble and the process of detecting a fluid break are essentially the same, except where indicated herein. As such, for simplicity this disclosure will often omit reference to a fluid break except where needed to distinguish from detection of a bubble.

In some embodiments, motion is detected by the optical tracking sensor and associated processor only when a leading edge of a bubble or fluid break crosses the optical tracking sensor and when a trailing edge of a bubble or fluid break crosses the optical tracking sensor. In some embodiments, no motion is detected by the optical tracking sensor and associated processor when there is no bubble passing the optical tracking sensor, when the leading edge is travelling across the optical tracking sensor except for the first instance of motion detected, or when the trailing edge is travelling across the optical tracking sensor except for the first instance of motion detected. In other words, there is a single detection of motion (a "blip") when the leading edge begins passing by the optical tracking sensor and a single detection of motion (another "blip") when the trailing edge begins passing by the optical tracking sensor, and no motion detected any other time.

In some embodiments, a bubble detection device receives an X-axis motion vector and/or a Y-axis motion vector when the leading edge of a bubble is detected. In some embodiments, a bubble detection device receives another X-axis motion vector and/or a Y-axis motion vector when the trailing edge of a bubble is detected.

In some embodiments, the X-axis motion vector and/or the Y-axis motion vector (of the leading edge and/or the trailing edge) may be a negative number. In some embodiments, when either the X-axis motion vector or the Y-axis motion vector is a negative number, this indicates that the fluid is flowing in a reverse direction.

In some embodiments, the motion vectors are used to calculate the movement of the leading edge (herein designated "movement (LE)"). In some embodiments, the motion vectors are used to calculate the movement of the trailing edge (herein designated "movement (TE)"). In some embodiments, the movement of the leading edge and the movement of the trailing edge are calculated using the equation: movement = sqrt ((dxₙ) ² + (dyₙ)²), where dxₙ is the X-axis motion vector of, respectively, the leading edge or the trailing edge and dyₙ is the Y-axis motion vector of, respectively, the leading edge or the trailing edge.

In some embodiments, the movement of the leading edge is used to calculate the net movement of the leading edge (herein designated "net movement (LE)"). In some embodiments, the movement of the trailing edge is used to calculate the net movement of the trailing edge (herein designated "net movement (TE)"). The net movement translates the motion vectors into a measurement based on the pixel pitch of the optical tracking sensor. In some embodiments, the net movement of the leading edge is calculated using the equation: net movement (LE) = movement (LE) * pixel pitch. In some embodiments, the net movement of the trailing edge is calculated using the equation: net movement (TE) = movement (TE) * pixel pitch.

In some embodiments, motion may be detected due to the presence of very small bubbles and/or very small contaminants that may be safely ignored. For example, in some embodiments, the smallest bubble of concern is 100 micrometer or 0.1 millimeter, and anything less than 100 micrometer may be ignored. As such, in some embodiments the net movement may be compared to a predetermined threshold to determine if the detected motion is due to something small enough to ignore. In some embodiments, the predetermined threshold is based on a multiple of the pixel pitch. For example, the predetermined threshold may be set as ten times the pixel pitch. Thus, in some embodiments, a bubble detection device determines if the net movement of the leading edge is greater than 10 * pixel pitch, and if not, the detected motion is ignored.

In some embodiments, the velocity of the detected bubble (designated "bubble_velocity") is determined. As described below, the velocity is used to determine the bubble length. In some embodiments, the velocity of the bubble (in millimeters per second (mm/sec)) is based on the velocity of the leading edge (designated herein as "velocity (LE)"), calculated using the equation: velocity (LE) = (movement (LE) * frame_refresh_rate) / 1000. In some embodiments, the velocity of the bubble (in mm/sec) is based on the velocity of the trailing edge (designated herein as "velocity (TE)"), calculated using the equation: velocity (TE) = (movement (TE) * frame_refresh_rate) / 1000. In some embodiments, the velocity of the bubble is based on averaging the velocity of the leading edge and the velocity of the trailing edge.

In some embodiments, a timer is started when a leading edge of a bubble is detected and the timer is stopped when a trailing edge of a bubble is detected. In some embodiments, the length of the bubble is calculated (in mm) using the equation: bubble_length = bubble_velocity * timer_value.

In some embodiments, a break in the fluid flow is identified by determining if the bubble length is greater than a predetermined multiple of a diameter of the tube. For example, a bubble may be considered a fluid break if the bubble length is greater than 20 times the diameter of the tube.

In some embodiments, a "tiny" bubble is identified by determining if the bubble length is less than a predetermined fraction of a diameter of the tube. For example, a bubble may be considered a tiny bubble if the bubble length is less than one-tenth the diameter of the tube. In some embodiments, a tiny bubble is ignored.

An estimate of an overall size of a detected bubble may be calculated using the equation: bubble_size = 0.5 * (net movement (LE) + net movement (TE)) * bubble_length.

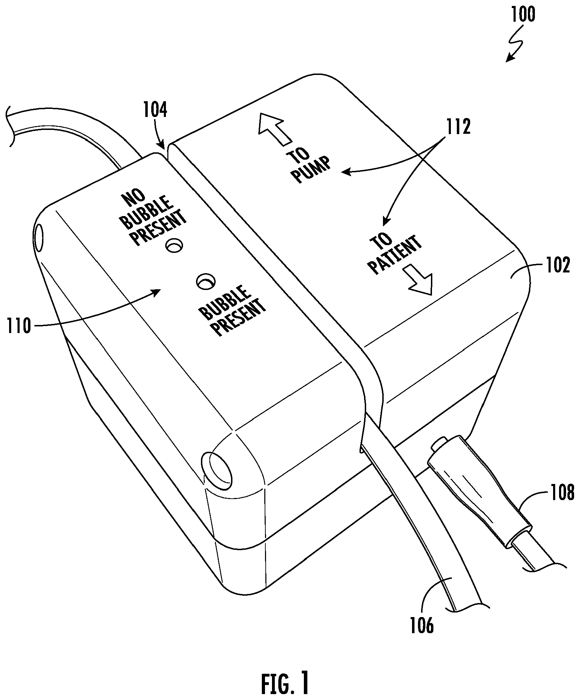

Referring now to FIG. 1, a perspective view of an example bubble detection device is illustrated in accordance with example embodiments of the present disclosure. As depicted in FIG. 1, in some embodiments a bubble detection device 100 comprises a housing 102 with a slot 104 defined therein for receiving a portion of a tube, such as an IV tube 106. In some embodiments, the housing 102 comprises a rectangular prism, as illustrated, although any suitable shape may be used. In some embodiments, the tube 106 fits snugly within the slot 104, with sufficient snugness such that the tube 106 is retained within the slot 104 but preferably without appreciably compressing the tube 106. In some embodiments, the housing 102 is constructed of any suitable material, such as any suitable plastic. In some embodiments, the housing 102 contains and protects internal components as described below.

In some embodiments, the bubble detection device 100 comprises a power cord 108 for receiving power from an AC outlet. In other embodiments, the bubble detection device 100 may be battery powered, with one or more batteries contained in the housing 102.

In some embodiments, the bubble detection device 100 comprises a mounting mechanism (not illustrated) for mounting the bubble detection device 100 in a convenient location, such as on the side of an IV pump.

In some embodiments, the bubble detection device 100 comprises an indicator light or the like to indicate whether or not a bubble has been detected. In the illustrated embodiment, indicator lights 110 include a green light indicating no bubble detected and a red light indicating bubble detected. In some embodiments, the bubble detection device 100 comprises an indicator light or the like (not illustrated) to indicate whether or not a reverse flow of the fluid has been detected.

In some embodiments, the bubble detection device 100 comprises an indicia to indicate the correct tube placement relative to a fluid source and a fluid destination. In the illustrated embodiment, indicia 112 indicates the correct tube placement relative to an IV pump (i.e., the fluid source) and to a patient (i.e., the fluid destination).

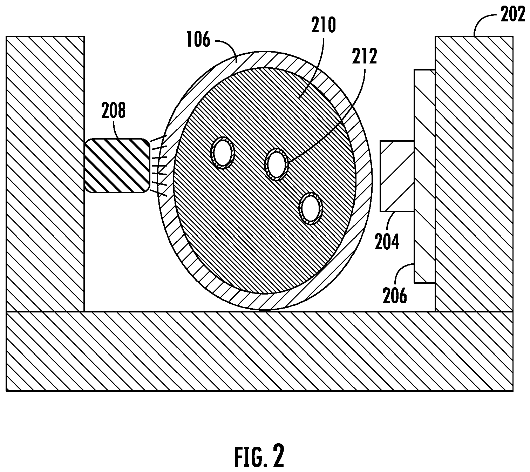

Referring now to FIG. 2, a simplified sectional view of a portion of the example bubble detection device 100 is illustrated. FIG. 2 illustrates some of the internal structure and components within the housing 102 and forming the slot 104. As seen in FIG. 2, an optical tracking sensor 204 is positioned on a circuit board 206 which is mounted to an internal structure 202 of the bubble detection device 100. As described above, the optical tracking sensor 204 comprises an array (e.g., 64x64) of individual sensing elements / pixels. The circuit board 206 comprises one or more additional electronic components to facilitate operation of the optical tracking sensor 204. In some embodiments, the circuit board 206 comprises a processor that controls operation of the optical tracking sensor 204, separate from a processor (not illustrated in FIG. 2) that controls the overall operation of the bubble detection device 100. In some embodiments, a single processor controls the operation of the optical tracking sensor and the overall operation of the bubble detection device.

An LED 208 is mounted to the internal structure 202 opposite the optical tracking sensor 204. When the tube 106 is inserted in the slot 104, the tube 106 is positioned between the optical tracking sensor 204 and the LED 208, as illustrated in FIG. 2, such that light emitted from the LED 208 is transmitted through the tube 106 and received by the optical tracking sensor 204 and processed as described herein. As illustrated in FIG. 2, the tube 106 contains a fluid 210 flowing therethrough. The fluid may contain one or more bubbles 212 to be detected.

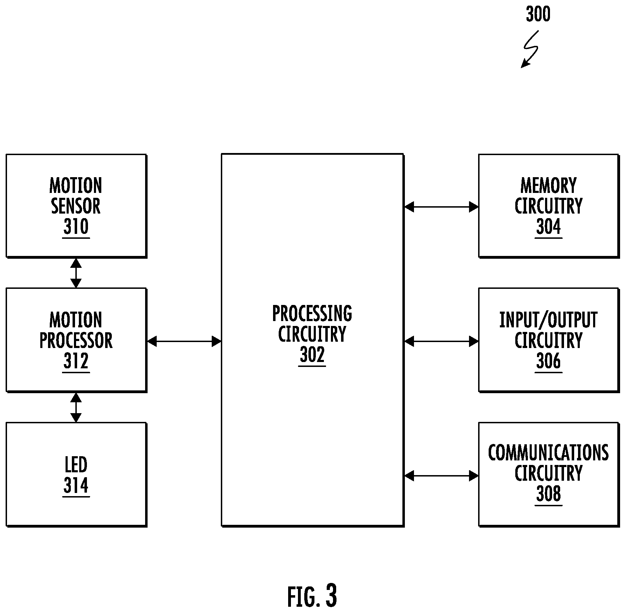

Referring now to FIG. 3, a block diagram of an example bubble detection device 300 in accordance with various embodiments of the present disclosure is provided. As depicted in FIG. 3, in some embodiments the bubble detection device 300 comprises a processor or processing circuitry 302, memory circuitry 304, input/output circuitry 306, communications circuitry 308, a motion sensor 310, a motion processor or processing circuitry 312, and an LED 314. In some embodiments, the bubble detection device 300 is configured to execute and perform the operations described herein.

Although components are described with respect to functional limitations, it should be understood that at least some of the particular implementations necessarily include the use of particular computing hardware. It should also be understood that in some embodiments certain of the components described herein include similar or common hardware. For example, in some embodiments two sets of circuitry both leverage use of the same processor(s), memory(ies), circuitry(ies), and/or the like to perform their associated functions such that duplicate hardware is not required for each set of circuitry.

Processing circuitry 302 may be embodied in a number of different ways. In various embodiments, the use of the terms "processor" or "processing circuitry" should be understood to include a single core processor, a multi-core processor, multiple processors internal to the bubble detection device 300, and/or one or more remote or "cloud" processor(s) external to the bubble detection device 300. In some example embodiments, processing circuitry 302 may include one or more processing devices configured to perform independently. Alternatively, or additionally, processing circuitry 302 may include one or more processor(s) configured in tandem via a bus to enable independent execution of operations, instructions, pipelining, and/or multithreading.

In an example embodiment, the processing circuitry 302 may be configured to execute instructions stored in the memory circuitry 304 or otherwise accessible to the processor. Alternatively, or additionally, the processing circuitry 302 may be configured to execute hard-coded functionality. As such, whether configured by hardware or software methods, or by a combination thereof, processing circuitry 302 may represent an entity (e.g., physically embodied in circuitry) capable of performing operations according to embodiments of the present disclosure while configured accordingly. Alternatively, or additionally, processing circuitry 302 may be embodied as an executor of software instructions, and the instructions may specifically configure the processing circuitry 302 to perform the various algorithms embodied in one or more operations described herein when such instructions are executed. In some embodiments, the processing circuitry 302 includes hardware, software, firmware, and/or a combination thereof that performs one or more operations described herein.

In some embodiments, the processing circuitry 302 (and/or co-processor or any other processing circuitry assisting or otherwise associated with the processor) is/are in communication with the memory circuitry 304 via a bus for passing information among components of the bubble detection device 300.

Memory or memory circuitry 304 may be non-transitory and may include, for example, one or more volatile and/or non-volatile memories. In some embodiments, the memory circuitry 304 includes or embodies an electronic storage device (e.g., a computer readable storage medium). In some embodiments, the memory circuitry 304 is configured to store information, data, content, applications, instructions, or the like, for enabling the bubble detection device 300 to carry out various operations and/or functions in accordance with example embodiments of the present disclosure.

Input/output circuitry 306 may be included in the bubble detection device 300. In some embodiments, input/output circuitry 306 may provide output to the user and/or receive input from a user. The input/output circuitry 306 may be in communication with the processing circuitry 302 to provide such functionality. The input/output circuitry 306 may comprise one or more user interface(s). In some embodiments, a user interface may include a display that comprises the interface(s) rendered as a web user interface, an application user interface, a user device, a backend system, or the like. In some embodiments, the input/output circuitry 306 also includes a keyboard, a mouse, a joystick, a touch screen, touch areas, soft keys a microphone, a speaker, or other input/output mechanisms. The processing circuitry 302 and/or input/output circuitry 306 may be configured to control one or more operations and/or functions of one or more user interface elements through computer program instructions (e.g., software and/or firmware) stored on a memory accessible to the processor (e.g., memory circuitry 304, and/or the like). In some embodiments, the input/output circuitry 306 includes or utilizes a user-facing application to provide input/output functionality to a computing device and/or other display associated with a user. In some embodiments, for example, the input/output circuitry 306 comprises indicator lights 110.

Communications circuitry 308 may be included in the bubble detection device 300. The communications circuitry 308 may include any means such as a device or circuitry embodied in either hardware or a combination of hardware and software that is configured to receive and/or transmit data from/to a network and/or any other device, circuitry, or module in communication with the bubble detection device 300. In some embodiments the communications circuitry 308 includes, for example, a network interface for enabling communications with a wired or wireless communications network. Additionally or alternatively, the communications circuitry 308 may include one or more network interface card(s), antenna(s), bus(es), switch(es), router(s), modem(s), and supporting hardware, firmware, and/or software, or any other device suitable for enabling communications via one or more communications network(s). In some embodiments, the communications circuitry 308 may include circuitry for interacting with an antenna(s) and/or other hardware or software to cause transmission of signals via the antenna(s) and/or to handle receipt of signals received via the antenna(s). In some embodiments, the communications circuitry 308 enables transmission to and/or receipt of data from a user device, one or more sensors, and/or other external computing device(s) in communication with the bubble detection device 300. In some embodiments, the communications circuitry 308 transmits (wired or wirelessly) information related to bubble detection to an IV pump, such that the IV pump stops pumping when a bubble is detected.

Motion sensor 310, along with motion processor 312, may be included in the bubble detection device 300. The motion sensor 310 comprises an optical tracking sensor having an array of individual sensing elements, as described above. Similar to the processing circuitry 302, the motion processor 312 may be embodied in a number of different ways, including but not limited to a single core processor, a multi-core processor, or multiple processors internal to the bubble detection device 300. In some embodiments, such as the embodiment of FIG. 3, the motion processor 312 is separate from the processing circuitry 302, as the bubble detection device 300 may use an off-the-shelf optical navigation system that includes both an optical tracking sensor and a motion processor (e.g., the PAA5100JE-Q from PixArt Imaging Inc. or the ADNS 3050-M from Avago Technologies Limited). In some embodiments (not illustrated), the functionality of the motion processor is included in the processing circuitry 302 and a separate motion processor is omitted.

LED 314 may be included in the bubble detection device 300. The LED 314 may be any suitable LED or other light source. In some embodiments, the LED is an infrared LED with a wavelength of around 950-980 nanometers.

In some embodiments, two or more of the sets of circuitry 302-308 are combinable. Alternatively, or additionally, one or more of the sets of circuitry 302-308 perform some or all of the operations and/or functionality described herein as being associated with another circuitry. In some embodiments, two or more of the sets of circuitry 302-308 are combined into a single module embodied in hardware, software, firmware, and/or a combination thereof.

While the description above provides an example bubble detection device 300, it is noted that the scope of the present disclosure is not limited to the description above. In some examples, an example bubble detection device 300 in accordance with the present disclosure may be in other forms. In some examples, an example bubble detection device 300 may comprise one or more additional and/or alternative elements, and/or may be structured differently than that illustrated in FIG. 3.

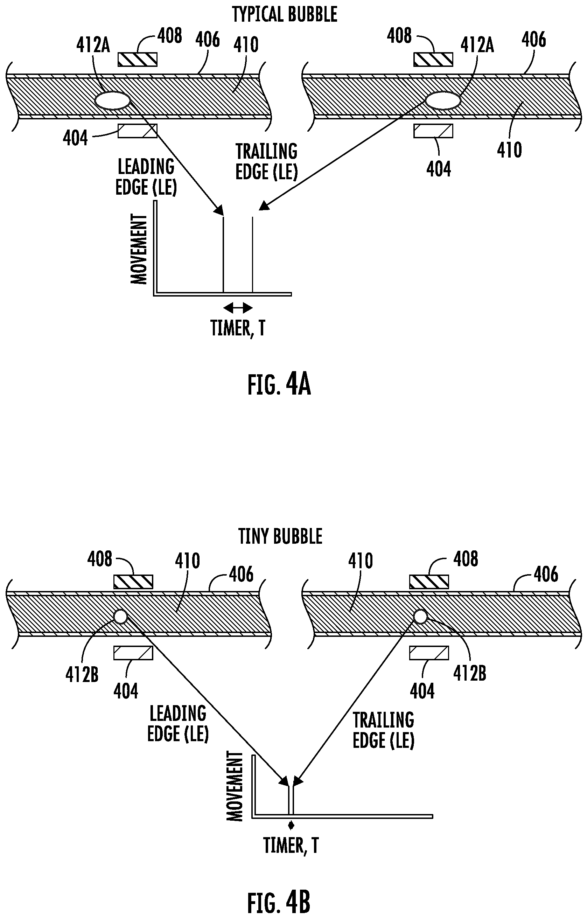

As described above, when the leading edge is first detected travelling across the optical tracking sensor, a single blip of motion is detected. Similarly, when the trailing edge is first detected travelling across the optical tracking sensor, a single blip of motion is detected. Referring now to FIGS. 4A-4C, example detections of, respectively, a typical bubble, a small bubble, and a break in the fluid flow are illustrated. In the graphs of FIGS. 4A-4C, the X-axis is time and the Y-axis is the magnitude of motion detected.

FIG. 4A illustrates an example detection of a typical (i.e., not tiny and not a break in the fluid) bubble 412A in a fluid 410 flowing through a tube 406 positioned between an optical tracking sensor 404 and a light source 408 (e.g., an LED). As seen in the graph in FIG. 4A, a first motion detection indication 414A corresponds with detection of the leading edge of the bubble 412A, and a second motion detection indication 416A corresponds with detection of the trailing edge of the bubble 412A. The distance between the first motion detection indication 414A and the second motion detection indication 416A is the amount of time (which is captured by the timer referenced above) between the detection of the leading edge and the detection of the trailing edge.

FIG. 4B illustrates an example detection of a tiny (e.g., having a length less than a predetermined fraction of a diameter of the tube) bubble 412B in a fluid 410 flowing through a tube 406 positioned between an optical tracking sensor 404 and a light source 408 (e.g., an LED). As seen in the graph in FIG. 4B a first motion detection indication 414B corresponds with detection of the leading edge of the bubble 412B, and a second motion detection indication 416B corresponds with detection of the trailing edge of the bubble 412B. The distance between the first motion detection indication 414B and the second motion detection indication 416B is the amount of time (which is captured by the timer referenced above) between the detection of the leading edge and the detection of the trailing edge.

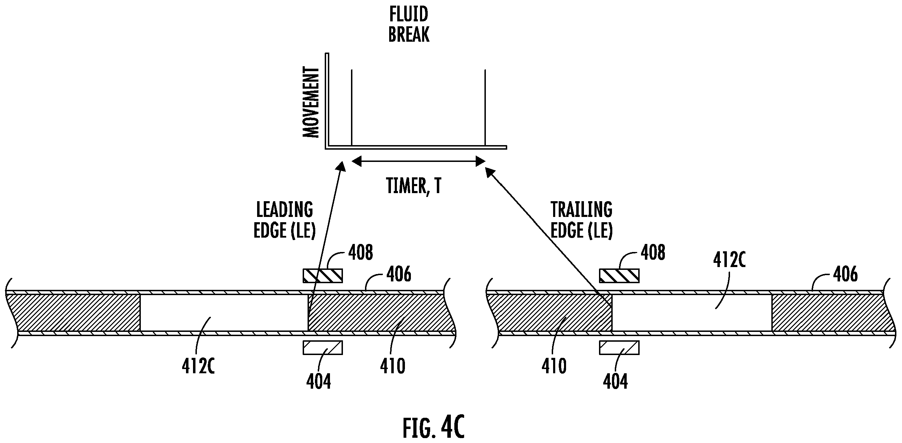

FIG. 4C illustrates an example detection of a fluid break (i.e., an air void in the fluid that is several times longer than the diameter of the tube) bubble 412C in a fluid 410 flowing through a tube 406 positioned between an optical tracking sensor 404 and a light source 408 (e.g., an LED). As seen in the graph in FIG. 4C a first motion detection indication 414C corresponds with detection of the leading edge of the bubble 412C, and a second motion detection indication 416C corresponds with detection of the trailing edge of the bubble 412C. The distance between the first motion detection indication 414C and the second motion detection indication 416C is the amount of time (which is captured by the timer referenced above) between the detection of the leading edge and the detection of the trailing edge.

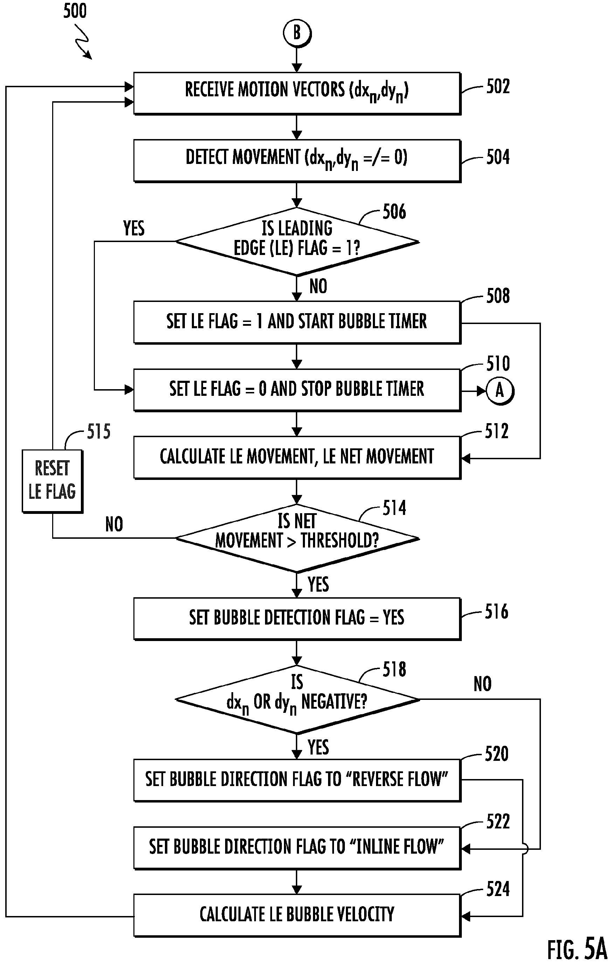

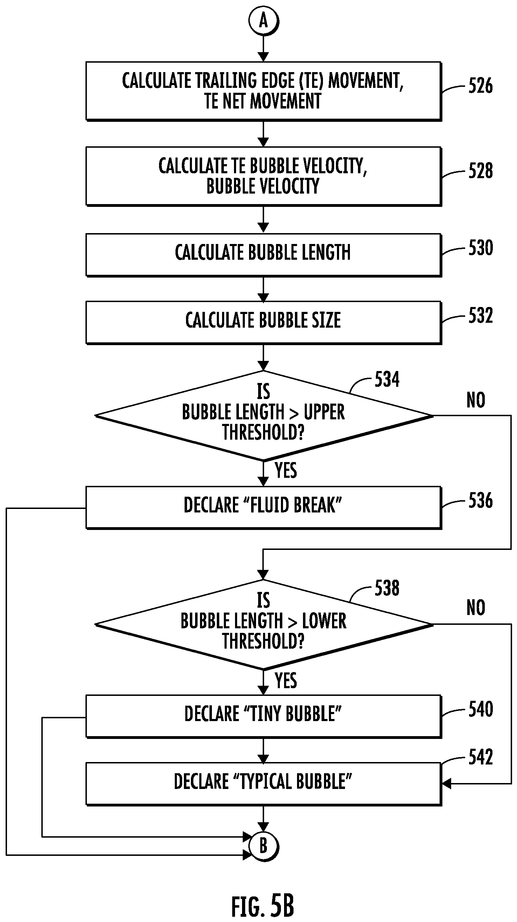

Reference will now be made to FIGS. 5A and 5B, which provides a flowchart illustrating example steps, processes, procedures, and/or operations in accordance with various embodiments of the present disclosure. Various methods described herein, including, for example, methods as shown in FIGS. 5A and 5B, may provide various technical benefits and improvements.

Referring now to FIGS. 5A and 5B, an example method 500 is illustrated. In some embodiments, the example method comprises a method for bubble detection in a fluid flow. At step/operation 502, a processor (such as, but not limited to, the processing circuitry 302 and/or motion processor 312 of the example bubble detection device 300 described above in connection with FIG. 3) receives motion vectors corresponding to the detection of motion within a fluid flowing in a tube. As described above, the motion vectors may comprise an X-axis motion vector (which may be expressed as dxₙ) and/or a Y-axis motion vector (which may be expressed as dyₙ).

At step/operation 504, a processor (such as, but not limited to, the processing circuitry 302 and/or motion processor 312 of the example bubble detection device 300 described above in connection with FIG. 3) determines if either of both of the motion vectors are non-zero, thereby indicating the detection of motion.

At step/operation 506, a processor (such as, but not limited to, the processing circuitry 302 and/or motion processor 312 of the example bubble detection device 300 described above in connection with FIG. 3) determines if a leading edge (LE) flag had previously been set to 1. If the LE flag had not previously been set to 1, in some embodiments this would indicate that a leading edge has not been detected (at least not in this cycle). If the LE flag had previously been set to 1, in some embodiments this would indicate that a leading edge has been detected (at least in this cycle).

If it is determined at step/operation 506 that the LE flag had not previously been set to 1, the method 500 proceeds to step/operation 508. In some embodiments, since the LE flag had not previously been set to 1 and motion was detected at step/operation 504, this indicates that the motion detected at step/operation 504 is a leading edge of a bubble or a fluid break. Thus, at step/operation 508, a processor (such as, but not limited to, the processing circuitry 302 and/or motion processor 312 of the example bubble detection device 300 described above in connection with FIG. 3) sets the LE flag to 1 and starts a bubble timer.

From step/operation 508, the method 500 proceeds to step/operation 512. At step/operation 512, a processor (such as, but not limited to, the processing circuitry 302 and/or motion processor 312 of the example bubble detection device 300 described above in connection with FIG. 3) calculates LE movement and LE net movement. As described above, in some embodiments, the movement of the leading edge is calculated using the equation: movement (LE) = sqrt ((dxₙ) ² + (dyₙ)²), where dxₙ is the X-axis motion vector of the leading edge and dyₙ is the Y-axis motion vector of the leading edge. Also as described above, in some embodiments, the net movement of the leading edge is calculated using the equation: net movement (LE) = movement (LE) * pixel pitch.

At step/operation 514, a processor (such as, but not limited to, the processing circuitry 302 and/or motion processor 312 of the example bubble detection device 300 described above in connection with FIG. 3) determines if the net movement of the leading edge is greater than a predetermined threshold. As described above, in some embodiments, the predetermined threshold is based on a multiple of the pixel pitch.

If it is determined at step/operation 514 that the net movement of the leading edge is not greater than a predetermined threshold, in some embodiments this means that whatever was detected is very small and can be ignored. The method 500 proceeds to step/operation 515, at which a processor (such as, but not limited to, the processing circuitry 302 and/or motion processor 312 of the example bubble detection device 300 described above in connection with FIG. 3) resets the LE flag to 0 and the method 500 returns to step/operation 502 to await detection of the next bubble or fluid break.

If it is determined at step/operation 514 that the net movement of the leading edge is greater than a predetermined threshold, in some embodiments this means that the detected motion is likely a bubble and the method 500 proceeds to step/operation 516. At step/operation 516, a processor (such as, but not limited to, the processing circuitry 302 and/or motion processor 312 of the example bubble detection device 300 described above in connection with FIG. 3) sets a bubble detection flag to "YES."

At step/operation 518, a processor (such as, but not limited to, the processing circuitry 302 and/or motion processor 312 of the example bubble detection device 300 described above in connection with FIG. 3) determines if either of the motion vectors is negative.

If it is determined at step/operation 518 that the one or both of the motion vectors are negative, in some embodiments this means that the fluid is flowing opposite the expected direction (i.e., reverse) and the method 500 proceeds to step/operation 520. At step/operation 520, a processor (such as, but not limited to, the processing circuitry 302 and/or motion processor 312 of the example bubble detection device 300 described above in connection with FIG. 3) sets a bubble direction flag to "REVERSE FLOW."

If it is determined at step/operation 518 that neither of the motion vectors are negative, in some embodiments this means that the fluid is flowing in the expected direction and the method 500 proceeds to step/operation 522. At step/operation 522, a processor (such as, but not limited to, the processing circuitry 302 and/or motion processor 312 of the example bubble detection device 300 described above in connection with FIG. 3) sets a bubble direction flag to "INLINE FLOW."

At step/operation 524, a processor (such as, but not limited to, the processing circuitry 302 and/or motion processor 312 of the example bubble detection device 300 described above in connection with FIG. 3) calculates the leading edge velocity. As described above, in some embodiments the velocity of the leading edge is calculated using the equation: velocity (LE) = (movement (LE) * frame_refresh_rate) / 1000.

In some embodiments, the method 500 then proceeds to step/operation 502 to await detection of a trailing edge, during which time the bubble timer is running. When subsequent motion vectors are received at step/operation 502 and movement is detected at step/operation 504, since the LE flag was previously set to 1 at step/operation 508, it would be determined at step/operation 506 that the LE flag = 1 and the method 500 proceeds to step/operation 510. Since the LE was previously set to 1, in some embodiments it is known that this subsequent detected motion is from a trailing edge (TE) of the same bubble.

At step/operation 510, a processor (such as, but not limited to, the processing circuitry 302 and/or motion processor 312 of the example bubble detection device 300 described above in connection with FIG. 3) sets the LE flag to 0, stops the bubble timer, and records the value of the bubble timer. The method 500 then proceeds to step/operation 526 in FIG. 5B.

At step/operation 526, a processor (such as, but not limited to, the processing circuitry 302 and/or motion processor 312 of the example bubble detection device 300 described above in connection with FIG. 3) calculates TE movement and TE net movement. As described above, in some embodiments, the movement of the trailing edge is calculated using the equation: movement (TE) = sqrt ((dxₙ) ² + (dyₙ)²), where dxₙ is the X-axis motion vector of the trailing edge and dyₙ is the Y-axis motion vector of the trailing edge. Also as described above, in some embodiments, the net movement of the trailing edge is calculated using the equation: net movement (TE) = movement (TE) * pixel pitch.

At step/operation 528, a processor (such as, but not limited to, the processing circuitry 302 and/or motion processor 312 of the example bubble detection device 300 described above in connection with FIG. 3) calculates the trailing edge velocity. As described above, in some embodiments the velocity of the trailing edge is calculated using the equation: velocity (TE) = (movement (TE) * frame_refresh_rate) / 1000. Also, at step/operation 528, a processor (such as, but not limited to, the processing circuitry 302 and/or motion processor 312 of the example bubble detection device 300 described above in connection with FIG. 3) calculates the velocity of the bubble. As described above, in some embodiments the velocity of the bubble is based on averaging the velocity of the leading edge and the velocity of the trailing edge.

At step/operation 530, a processor (such as, but not limited to, the processing circuitry 302 and/or motion processor 312 of the example bubble detection device 300 described above in connection with FIG. 3) calculates the length of the detected bubble. As described above, in some embodiments the length of the bubble is calculated using the equation: bubble_length = bubble_velocity * timer_value.

At step/operation 532, a processor (such as, but not limited to, the processing circuitry 302 and/or motion processor 312 of the example bubble detection device 300 described above in connection with FIG. 3) calculates an estimate of the size of the detected bubble. As described above, in some embodiments an estimate of an overall size of a detected bubble may be calculated using the equation: bubble_size = 0.5 * (net movement (LE) + net movement (TE)) * bubble_length.

At step/operation 534, a processor (such as, but not limited to, the processing circuitry 302 and/or motion processor 312 of the example bubble detection device 300 described above in connection with FIG. 3) determines if the bubble length is greater than a predetermined upper threshold. As described above, in some embodiments a break in the fluid flow is identified by determining if the bubble length is greater than a predetermined multiple of a diameter of the tube. For example, a bubble may be considered a fluid break if the bubble length is greater than 20 times the diameter of the tube. Thus, in some embodiments it is determined at step/operation 534 if the bubble length is greater than 20 times the diameter of the tube.

If it is determined at step/operation 534 that the bubble length is greater than a predetermined upper threshold, the method 500 proceeds to step/operation 536 and a "FLUID BREAK" is declared. The method 500 then returns to step/operation 502 to await detection of the next bubble or fluid break.

If it is determined at step/operation 534 that the bubble length is not greater than a predetermined upper threshold, the method 500 proceeds to step/operation 538.

At step/operation 538, a processor (such as, but not limited to, the processing circuitry 302 and/or motion processor 312 of the example bubble detection device 300 described above in connection with FIG. 3) determines if the bubble length is less than a predetermined lower threshold. As described above, in some embodiments a tiny bubble in the fluid flow is identified by determining if the bubble length is less than a predetermined fraction of a diameter of the tube. For example, a bubble may be considered a tiny bubble if the bubble length is less than one-tenth the diameter of the tube. Thus, in some embodiments it is determined at step/operation 538 if the bubble length is less than one-tenth the diameter of the tube.

If it is determined at step/operation 538 that the bubble length is less than a predetermined lower threshold, the method 500 proceeds to step/operation 540 and a "TINY BUBBLE" is declared. The method 500 then returns to step/operation 502 to await detection of the next bubble or fluid break.

If it is determined at step/operation 538 that the bubble length is not less than a predetermined lower threshold, the method 500 proceeds to step/operation 542 and a "TYPICAL BUBBLE" is declared. The method 500 then returns to step/operation 502 to await detection of the next bubble or fluid break.

In some embodiments, the method 500 repeats as long as a corresponding fluid delivery device is pumping fluid.

Although not illustrated in FIGS. 5A and 5B, in various embodiments of the present disclosure there may be one or more predetermined actions that are taken in response to one or more determinations and/or steps/operations of a bubble detection method. For example, in some embodiments when a bubble is detected, an indicator (such as indicator lights 110) may be activated to indicate to a user the presence of a bubble. As another example, in some embodiments when a reverse fluid flow is detected, an alarm may be sounded and/or a signal may be sent to a fluid delivery device to cause the fluid delivery device to stop pumping the fluid. In yet another example, in some embodiments when a break in the fluid flow is detected, an alarm may be sounded and/or a signal may be sent to a fluid delivery device to cause the fluid delivery device to stop pumping the fluid.

Operations and processes described herein support combinations of means for performing the specified functions and combinations of operations for performing the specified functions. It will be understood that one or more operations, and combinations of operations, may be implemented by special purpose hardware-based computer systems which perform the specified functions, or combinations of special purpose hardware and computer instructions.

In some example embodiments, certain ones of the operations herein may be modified or further amplified as described below. Moreover, in some embodiments additional optional operations may also be included. It should be appreciated that each of the modifications, optional additions or amplifications described herein may be included with the operations herein either alone or in combination with any others among the features described herein.

The foregoing method and process descriptions are provided merely as illustrative examples and are not intended to require or imply that the steps of the various embodiments must be performed in the order presented. As will be appreciated by one of skill in the art the order of steps in the foregoing embodiments may be performed in any order. Words such as "thereafter," "then," "next," and similar words are not intended to limit the order of the steps; these words are simply used to guide the reader through the description of the methods. Further, any reference to claim elements in the singular, for example, using the articles "a," "an" or "the," is not to be construed as limiting the element to the singular and may, in some instances, be construed in the plural.

While various embodiments in accordance with the principles disclosed herein have been shown and described above, modifications thereof may be made by one skilled in the art without departing from the teachings of the disclosure. The embodiments described herein are representative only and are not intended to be limiting. Many variations, combinations, and modifications are possible and are within the scope of the disclosure. Alternative embodiments that result from combining, integrating, and/or omitting features of the embodiment(s) are also within the scope of the disclosure. Accordingly, the scope of protection is not limited by the description set out above, but is defined by the claims which follow, that scope including all equivalents of the subject matter of the claims. Each and every claim is incorporated as further disclosure into the specification and the claims are embodiment(s) of the present disclosure. Furthermore, any advantages and features described above may relate to specific embodiments but shall not limit the application of such issued claims to processes and structures accomplishing any or all of the above advantages or having any or all of the above features.

In addition, the section headings used herein are provided for consistency with the suggestions under 37 C.F.R. § 1.77 or to otherwise provide organizational cues. These headings shall not limit or characterize the disclosure set out in any claims that may issue from this disclosure. For instance, a description of a technology in the "Background" is not to be construed as an admission that certain technology is prior art to any disclosure in this disclosure. Neither is the "Summary" to be considered as a limiting characterization of the disclosure set forth in issued claims. Furthermore, any reference in this disclosure to "disclosure" or "embodiment" in the singular should not be used to argue that there is only a single point of novelty in this disclosure. Multiple embodiments of the present disclosure may be set forth according to the limitations of the multiple claims issuing from this disclosure, and such claims accordingly define the disclosure, and their equivalents, which are protected thereby. In all instances, the scope of the claims shall be considered on their own merits in light of this disclosure but should not be constrained by the headings set forth herein.

Also, systems, subsystems, apparatuses, techniques, and methods described and illustrated in the various embodiments as discrete or separate may be combined or integrated with other systems, modules, techniques, or methods without departing from the scope of the present disclosure. Other devices or components shown or discussed as coupled to, or in communication with, each other may be indirectly coupled through some intermediate device or component, whether electrically, mechanically, or otherwise. Other examples of changes, substitutions, and alterations are ascertainable by one skilled in the art and could be made without departing from the scope disclosed herein.

Many modifications and other embodiments of the disclosure set forth herein will come to mind to one skilled in the art to which these embodiments pertain having the benefit of teachings presented in the foregoing descriptions and the associated figures. Although the figures only show certain components of the apparatuses and systems described herein, various other components may be used in conjunction with the components and structures disclosed herein. Therefore, it is to be understood that the disclosure is not to be limited to the specific embodiments disclosed and that modifications and other embodiments are intended to be included within the scope of the appended claims. For example, the various elements or components may be combined, rearranged, or integrated in another system or certain features may be omitted or not implemented. Moreover, the steps in any method described above may not necessarily occur in the order depicted in the accompanying drawings, and in some cases one or more of the steps depicted may occur substantially simultaneously, or additional steps may be involved. Although specific terms are employed herein, they are used in a generic and descriptive sense only and not for purposes of limitation.

## Claims

1. A bubble detection device comprising:
a light source adapted to be positioned to shine light through a tube through which a fluid flows;
a light sensor adapted to be positioned on an opposite side of the tube from the light source and to receive light from the light source transmitted through the tube; and
one or more processors that (i) detect a first motion across the light sensor, the first motion corresponding to a leading edge of a bubble in the fluid, (ii) detect a second motion across the light sensor, the second motion corresponding to a trailing edge of the bubble in the fluid, and (iii) calculate a length of the bubble based on (a) a velocity of the bubble and (b) a time between detection of the first motion and detection of the second motion.

2. The bubble detection device of claim 1, wherein the light is received by the light sensor and processed by the one or more processors at a frame refresh rate; and
wherein the one or more processors calculate the velocity of the bubble based on an amount of movement of the bubble across the light sensor in one frame multiplied by the frame refresh rate.

3. The bubble detection device of claim 2, wherein the one or more processors calculates the velocity of the bubble based on (1) an amount of movement of the leading edge across the light sensor in the detected first motion multiplied by the frame refresh rate, (2) an amount of movement of the trailing edge across the light sensor in the detected second motion multiplied by the frame refresh rate, or (3) an average of the amount of movement of the leading edge across the light sensor in the detected first motion multiplied by the frame refresh rate and the amount of movement of the trailing edge across the light sensor in the detected second motion multiplied by the frame refresh rate.

4. The bubble detection device of claim 1, wherein the first motion is expressed as an X-axis motion vector and/or a Y-axis motion vector; and
wherein the one or more processors detect a reverse flow of the fluid based on a negative value of the X-axis motion vector and/or a negative value of the Y-axis motion vector.

5. The bubble detection device of claim 1, wherein the one or more processors start a timer when the first motion is detected and stop the timer when the second motion is detected.

6. The bubble detection device of claim 1, wherein the one or more processors calculate an estimate of a size of the bubble based on 0.5 * (an amount of movement of the leading edge across the light sensor in the detected first motion + an amount of movement of the trailing edge across the light sensor in the detected second motion) * the length of the bubble.

7. The bubble detection device of claim 1, wherein the one or more processors determine if a break in the fluid flow has occurred by determining if the bubble length is greater than a predetermined multiple of a diameter of the tube.

8. The bubble detection device of claim 1, wherein the one or more processors determine if the bubble is classified as a small bubble by determining if the bubble length is less than a predetermined fraction of a diameter of the tube.

9. A method of detecting a bubble in a fluid flowing through a tube, the method comprising:
shining light from a light source positioned to shine the light through the tube;
receiving, by a light sensor positioned on an opposite side of the tube from the light source, light from the light source transmitted through the tube;
detecting, by one or more processors, a first motion across the light sensor, the first motion corresponding to a leading edge of a bubble in the fluid;
detecting, by the one or more processors, a second motion across the light sensor, the second motion corresponding to a trailing edge of the bubble in the fluid; and
calculating, by the one or more processors, a length of the bubble based on (a) a velocity of the bubble and (b) a time between detection of the first motion and detection of the second motion.

10. The method of claim 9, wherein the light is received by the light sensor and processed by the one or more processors at a frame refresh rate; and
wherein the method further comprises calculating, by the one or more processors, the velocity of the bubble based on an amount of movement of the bubble across the light sensor in one frame multiplied by the frame refresh rate.

11. The method of claim 10, further comprising:
calculating, by the one or more processors, the velocity of the bubble based on (1) an amount of movement of the leading edge across the light sensor in the detected first motion multiplied by the frame refresh rate, (2) an amount of movement of the trailing edge across the light sensor in the detected second motion multiplied by the frame refresh rate, or (3) an average of the amount of movement of the leading edge across the light sensor in the detected first motion multiplied by the frame refresh rate and the amount of movement of the trailing edge across the light sensor in the detected second motion multiplied by the frame refresh rate.

12. The method of claim 9, wherein the first motion is expressed as an X-axis motion vector and/or a Y-axis motion vector; and
wherein the method further comprises detecting, by the one or more processors, a reverse flow of the fluid based on a negative value of the X-axis motion vector and/or a negative value of the Y-axis motion vector.

13. The method of claim 9, further comprising:
calculating, by the one or more processors, an estimate of a size of the bubble based on 0.5 * (an amount of movement of the leading edge across the light sensor in the detected first motion + an amount of movement of the trailing edge across the light sensor in the detected second motion) * the length of the bubble.

14. The method of claim 9, further comprising:
determining, by the one or more processors, if a break in the fluid flow has occurred by determining if the bubble length is greater than a predetermined multiple of a diameter of the tube.

15. The method of claim 9, further comprising:
determining, by the one or more processors, if the bubble is classified as a small bubble by determining if the bubble length is less than a predetermined fraction of a diameter of the tube.
